# EUROPÄISCHE PATENTSCHRIFT

(11) **EP 3 319 585 B1**
(45) Veröffentlichungstag und Bekanntmachung des Hinweises auf die Patenterteilung: **12.02.2020**
(21) Anmeldenummer: 16731134.9
(22) Anmeldetag: 21.06.2016
(51) Int. Cl.: A61K 8/44, A61Q 1/14, A61K 8/60, A61K 8/90, A61K 8/02

(54) **REINIGUNGSTÜCHER GETRÄNKT MIT TRÄNKUNGSMITTELN BASIEREND AUF EINER MIZELLENTECHNOLOGIE**
CLEANSING WIPES IMPREGNATED WITH A MICELLE FORMING FORMULATION
CHIFFONS DE NETTOYAGE AVEC UNE FORMULATION QUI FORME DES MICELLES

(30) Priorität: 09.07.2015 DE 102015212822
(43) Veröffentlichungstag der Anmeldung: 16.05.2018
(73) Patentinhaber: Beiersdorf AG, 20253 Hamburg (DE)
(72) Erfinder: VIETENSE, Christin, 22177 Hamburg (DE); SCHÄFER, Jessica, 22113 Oststeinbek (DE)
(86) Internationale Anmeldenummer: PCT/EP2016/064292
(87) Internationale Veröffentlichungsnummer: WO 2017/005485

(56) Entgegenhaltungen:
- DE-A1- 10 157 543
- DATABASE GNPD [Online] MINTEL; April 2014 (2014-04), "3in1 Cleansing Fluid", XP002759955, Database accession no. 2368616

## Beschreibung

Die vorliegende Erfindung betrifft Tücher getränkt mit Tränkungsmitteln, die sich dadurch auszeichnen, dass sie auf einer Mizellentechnologie basieren.

Die Verwendung von Make-up zur Verschönerung der äußeren Erscheinung, insbesondere des Gesichts, ist ein Phänomen, das schon lange zu beobachten ist. Bereits im alten Ägypten wurden Kajal, Lidschatten, Rouge und Lippenstift verwendet und zwar von Männern und Frauen. Neben einer Schutzfunktion hatte das Schminken auch kosmetische Effekte. Make-up wurde einfach um der Schönheit willen getragen. Dies gilt auch heute noch.

Das verwendete Make-up muss auch wieder entfernt werden. Zur Entfernung von Make-up aus dem Gesichtsbereich gibt es eine besondere Gruppe von Hautreinigungsprodukten, nämlich Gesichtsreinigungsprodukte. Da die Gesichtshaut besonders empfindlich ist, werden für die Gesichtsreinigung besonders milde und die Haut nicht reizende Produkte eingesetzt.

Im Bereich der dekorativen Kosmetik finden heutzutage eine Vielzahl von unterschiedlichen Stoffen Anwendung. Als Farbstoffe werden neben anorganischen Pigmenten wie Silikaten [Magnesiumsilikat (Talkum), Aluminiumsilikat (Kaolin)] und Metalloxiden (Chrom-, Eisen-, Mangan-, Titan- und Zinkoxiden) organische Farbpigmente eingesetzt. Als Bindemittel finden unter anderem Stearinsäureester, Lanolinalkohol und -acetat Verwendung. In vielen Formulierungen werden Wachse wie Bienenwachs oder Carnaubawachs und Öle wie Paraffinöle, Silikonöle oder Ricinusöl eingesetzt. Des Weiteren können dekorative Kosmetika Konservierungsstoffe, Antioxidantien, Verdickungsmittel und andere Zusätze enthalten.

Um diese Vielzahl völlig unterschiedlicher Stoffe von der Haut zu entfernen, bedarf es entsprechender kosmetischer Reinigungsmittel. Sie müssen unpolare Verbindungen wie Wachse, Öle und Silikonverbindungen lösen und gleichzeitig die schwerlöslichen Pigmente wie Talkum oder Titandioxid aufnehmen. Dies gilt insbesondere für Wimperntusche, Mascara, Lidschatten und Kajalstifte. Andererseits müssen sie so hautverträglich wie möglich sein, um bei den Anwendern keine Hautrötungen oder Schleimhautirritationen auszulösen.

Eine besondere Ausführungsform kosmetischer Reinigungsmittel stellen die Tücher dar. Diese können mit einer Reinigungszubereitung getränkt sein, welche die mechanische Entfernung des Schmutzes von der Haut unterstützt. Kommerziell erhältliche mit Reinigungsmitteln getränkte Tücher haben darüber hinaus den Vorteil, dass in ihnen die Reinigungszubereitung bereits in der richtigen Menge vorgegeben ist. Darüber hinaus haben Reinigungstücher den Vorteil, dass sie einfach mitgenommen werden können. Das bedeutet, dass sie zu jeder Gelegenheit zu Reinigungszwecken zur Verfügung stehen, beispielsweise unterwegs auf Reisen oder auch bei der Arbeit. Außerdem vermeiden die Reinigungstücher die Nachteile von in Flaschen aufbewahrten Reinigungsmitteln, deren Verpackung zerbrechen und deren Inhalt "auslaufen" kann.

Im Stand der Technik gibt es eine Vielzahl von Dokumenten, die verschiedenartige Reinigungstücher mit unterschiedlichen Tränkungsmitteln offenbaren.

DE 10157543 offenbart Emulsions-getränkte Tücher, wobei die Emulsion Chitosan und/oder Lecithin enthält. Dadurch kann der Gehalt an Emulgatoren und Konservierungsmitteln reduziert werden.

DE 10219638 beschreibt ebene, gelochte Reinigungstücher, die mit einem emulsions-artigen Tränkungsmittel getränkt sind. Diese Tücher bewirken eine sanfte Reinigung, insbesondere Gesichtsreinigung.

Im Dokument EP 1496853 werden Reinigungstücher beschrieben, die mit einem Tränkungsmittel getränkt sind, das Stärke-haltige Verbindungen enthält. Die Einarbeitung dieser Stärke-haltigen Verbindungen führt zu einem besonders angenehmen, glatten, samtigen Hautgefühl.

EP 1405632 offenbart die Einarbeitung von Zinkoxidpartikeln einer bestimmten Größe in die Tränkungsmittel und die Möglichkeit diese Tränkungsmittel auf die Tücher aufzubringen.

Im Dokument DE 102012200383 werden Reinigungstücher offenbart, die eine raue Oberfläche aufweisen. Dies führt zu einem Peelingeffekt. Um die Stärke des Peelingeffekts steuern zu können, werden verschiedene Mengen des Tränkungsmittels aufgebracht.

Im Dokument DE 202004007851 werden gefärbte Reinigungstücher und Tränkungsmittel beschrieben. Die eingesetzten Farbstoffe und Tränkungsmittel sind so gewählt, dass die Farbe auf dem Tuch verbleibt und nicht in das Tränkungsmittel übertritt.

Lipidhaltige Zusammensetzungen zur Tränkung von Tüchern werden im Dokument WO 2005/044220 A1 offenbart. Diese Tränkungsmittel bewirken, dass Reinigungsartikel getränkt mit diesen Mitteln neben einem hohen Reinigungsvermögen auch ein hohes Rückfettungsvermögen aufweisen.

Klassische Reinigungstücher sind also häufig mit Tränkungsmitteln getränkt, die auf Tensidfreien Zubereitungen basieren. Dies kann dazu führen, dass die Reinigungsleistung gemindert ist. Mit derartig getränkten Reinigungstüchern ist insbesondere die Entfernung von wasserfestem Make-up und hier insbesondere diejenige von wasserfester Mascara problematisch. Öl-haltige Reinigungstücher werden daher häufig zur Entfernung von wasserfesten Make-up-Zubereitungen eingesetzt. Diese Öl-haltigen Reinigungstücher haben den Nachteil, dass Öle aufgrund ihrer unterschiedlich hohen Spreitfähigkeit ins Auge gelangen können. Je dünnflüssiger ein Öl ist, desto höher ist im Allgemeinen das Spreitungsvermögen und desto leichter kann es ins Auge gelangen. Mit den Ölen können aber auch andere, in der Zubereitung enthaltene, Rohstoffe ins Auge gelangen. Dies führt zu einer verminderten Verträglichkeit dieser Produkte. Des Weiteren bleibt bei der Verwendung Öl-haltiger Reinigungstücher auf der Haut oft ein öliger Rückstand zurück, der von vielen Anwendern als unangenehm empfunden wird.

Wässrig getränkte Tücher bedürfen einer hinreichenden Konservierung, damit keine Keime in den Augenbereich eingeschleppt werden. Neben anderen Organen zeichnet sich das Auge durch eine reduzierte Aktivierbarkeit des spezifischen und des unspezifischen Immunsystems aus. Dies wird als Immunprivileg bezeichnet. Ebenso ist bei der Auswahl der Konservierungsstoffe zu beachten, dass diese Stoffe ein möglichst geringes Reizpotential für den Augenbereich haben.

Es war daher die Aufgabe der vorliegenden Erfindung, die Mängel des Standes der Technik zu beseitigen und mit wässrigen Reinigungsmitteln getränkte Tücher zu entwickeln, deren Reinigungskraft derart verbessert ist, dass wasserfeste Make-up-Zubereitungen und insbesondere wasserfeste Mascara-Zubereitungen problemlos entfernt werden können. Dabei sollen die Produkte hinreichend konserviert und dabei doch gut verträglich sein.

Überraschend werden die genannten Aufgaben durch Tücher, getränkt mit einem wässrigen Tränkungsmittel gelöst, dass wenigstens ein Blockpolymer, eine Mischung aus wenigstens einem Natrium Acylglutamat und wenigstens einem Alkylpolyglycosid und Alkohol denat. enthält und wobei das Gewichtsverhältnis von dem wenigstens einem Blockpolymer zu der Mischung aus dem wenigstens einen Natrium Acylglutamat und den wenigstens einem Alkylpolyglycosid 3,63 : 1 bis 0,28 : 1 beträgt.

Die erfindungsgemäße Mischung ausgewählter Tenside enthält zum einen wenigstens ein Natrium Acylglutamat. Bevorzugt werden folgende Natrium Acylglutamate eingesetzt: Natrium/Dinatrium Cocoylglutamate, Natrium/Dinatrium Lauroylglutamate sowie Natrium/Dinatrium Myristoylglutamate. Ganz besonders bevorzugt ist es, wenn als Natrium Acylglutamat Dinatrium Cocoylglutamat eingesetzt wird. Dinatrium Cocoylglutamat ist beispielsweise erhältlich unter der Handelsbezeichnung Protelan AGL 95/C von der Firma Zschimmer & Schwarz.

Natrium Acylglutamate sind in den erfindungsgemäßen Tränkungsmitteln mit einem Gehalt von 0,001 bis 1 Gew. %, bevorzugt von 0,003 bis 0,75 Gew. %, besonders bevorzugt von 0,005 bis 0,5 Gew. %, bezogen auf das Gesamtgewicht des Tränkungsmittels, enthalten.

Die erfindungsgemäße Mischung ausgewählter Tenside enthält zum anderen wenigstens ein Alkylpolyglycosid. Bevorzugt werden folgende Alkylpolyglycoside eingesetzt: Decylglucoside, Laurylglucoside sowie Coco Glucoside. Ganz besonders bevorzugt ist es, wenn als Alkylpolyglycosid Decylpolyglucose eingesetzt wird. Decylpolyglucose ist beispielsweise erhältlich unter der Handelsbezeichnung Plantacare 2000 UP von der Firma BASF Personal Care.

Alkylpolyglycoside sind in den erfindungsgemäßen Tränkungsmitteln mit einem Gehalt von 0,01 bis 1 Gew. %, bevorzugt von 0,025 bis 0,8 Gew. %, besonders bevorzugt von 0,05 bis 0,6 Gew. %, bezogen auf das Gesamtgewicht des Tränkungsmittels, enthalten.

In den erfindungsgemäßen Tränkungsmitteln beträgt das Gewichtsverhältnis der Natrium Acylglutamate zu den Alkylpolyglycosiden 30:1 bis 0,001:1, bevorzugt 20:1 bis 0,05:1.

Die erfindungsgemäßen Tränkungsmittel enthalten wenigstens ein Blockpolymer. Blockpolymere sind Copolymere aus Ethylenoxid- und Propylenoxid-Einheiten mit der Bezeichnung Poloxamer. Sie werden durch die Formel

HO(C₂H₄O)ₐ(C₃H₆O)_{b}(C₂H₄O)ₐH

beschrieben, wobei b die Werte von 15 bis 67 und a die Werte 1 bis 130 einnehmen kann. Der Anteil der a-Einheiten kann von 20 bis 90 % des Poloxamers ausmachen. Folgende Poloxamere kommen vorzugsweise zum Einsatz, Poloxamer 101, Poloxamer 124 und Poloxamer 184. Besonders bevorzugt ist der Einsatz von Poloxamer 124. Diese Verbindung ist beispielsweise erhältlich unter der Handelsbezeichnung Synperonic PE/L44 bei der Firma Croda (ICI Chemicals & Polymers).

Die Blockpolymere liegen mit einem Gehalt von 0,1 bis 8 Gew. %, bevorzugt von 0,2 bis 5 Gew. %, besonders bevorzugt von 0,25 bis 4 Gew. %, bezogen auf das Gesamtgewicht des Tränkungsmittels, in den erfindungsgemäßen Tränkungsmitteln vor.

In den erfindungsgemäßen Tränkungsmitteln beträgt das Gewichtsverhältnis von dem wenigstens einen Blockpolymer zu der Mischung aus dem wenigstens einen Natrium Acylglutamat und dem wenigstens einen Alkylpolyglycosid 3,63: 1 bis 0,28:1.

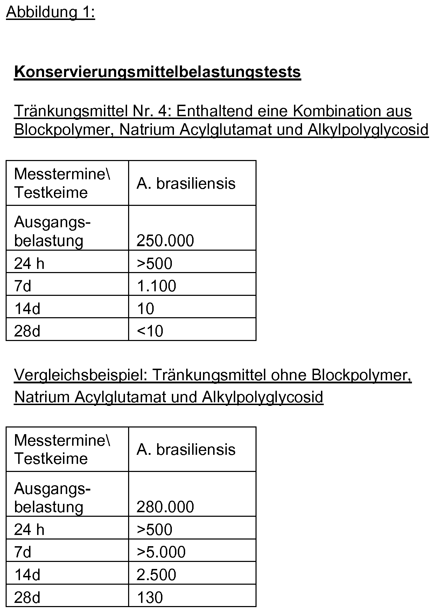

Die erfindungsgemäße Mischung von Tensiden mit Blockpolymeren in Tränkungsmedien für Reinigungstücher hat dazu geführt, dass eine stabile Konservierung bei guter Verträglichkeit erreicht werden konnte. Die erfindungsgemäße Mischung wurde mit dem Keim A. brasiliensis in Kontakt gebracht. Bereits nach 24 h waren kaum noch Keime nach zuweisen. Der Keim
A. brasiliensis, der bei Verkeimungen von Reinigungstüchern häufig nachzuweisen ist, wird durch die milde, aber effektive Konservierung der erfindungsgemäßen Zubereitung gut beherrscht. In der erfindungsgemäßen Zubereitung sind nach 24 h mehr als 99,8 % der A. brasiliensis-Keime abgetötet. Die Ergebnisse sind in der Abbildung 1 dargestellt.

Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Als Konservierungsmittel können vorteilhaft Phenoxyethanol, Methyl-, Ethyl-, Propylparaben, Polyaminopropyl Biguanide, Potassium Sorbate, Benzethonium Chloride, Sodium Benzoate, Sodium Salicylate und Benzyl Alcohol eingesetzt werden.

Als Stabilisatoren sind im Sinne der vorliegenden Erfindung diejenigen Substanzen zu verstehen, die nicht in der Liste der zugelassenen Konservierungsstoffe (Kosmetikverordnung Anlage 6; Verordnung (EG) Nr. 1223/2009, Anhang V) aufgeführt sind, gleichwohl aber eine stabilisierende Wirkung haben und/oder im gemeinsamen Einsatz mit Konservierungsmitteln die Stabilität der Zubereitung 36 Monate fördern.

Als Stabilisatoren sind folgende Verbindungen vorteilhaft: Alcohol Denat., Propylene Glycol, Ethylhexylglycerin, 1,2-Hexanediol, Trisodium EDTA, Methylpropanediol, Butylene Glycol, Caprylyl Glycol, Piroctone Olamine und Pentylene Glycol.

Bevorzugt kommen die Verbindungen Phenoxyethanol, Methyl-, Ethyl-, Propylparaben, Alcohol Denat., Ethylhexylglycerin, 1,2-Hexanediol, Polyaminopropyl Biguanide, Trisodium EDTA, Caprylyl Glycol und Piroctone Olamine und ganz besonders bevorzugt sind die Verbindungen Phenoxyethanol, Alcohol Denat., Ethylhexylglycerin, 1,2-Hexanediol und Trisodium EDTA in den erfindungsgemäßen Tränkungsmitteln zum Einsatz.

Die Konservierungsmittel und/oder Stabilisatoren liegen mit einem Gehalt von 0,0001 bis 10 Gew. %, bevorzugt von 0,0005 bis 8 Gew. %, besonders bevorzugt von 0,001 bis 7 Gew. % in den Tränkungsmitteln vor.
Überraschenderweise wurde gefunden, dass Alcohol Denat. nicht nur als Stabilisator eingesetzt werden kann, sondern auch die Feuchthaltung der Tücher positiv beeinflusst. Ein weiterer Vorteil ist, dass die Tücher bei einem geringen Tränkungsgrad sehr feucht sind und auch länger feucht erscheinen. Das erfindungsgemäße Tränkungsmittel enthält Alkohol denat.

Tenside sind amphiphile Stoffe, die organische, unpolare Substanzen in Wasser lösen können. Sie zeichnen sich durch ein ambivalentes Verhalten gegenüber Wasser und Lipiden aus. Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und-lösung, ein leichtes Abspülen und - je nach Wunsch - auch für Schaumregulierung. Damit ist die Grundlage für die Schmutzentfernung lipidhaltiger Verschmutzungen gegeben.

In der vorliegenden Erfindung werden die Tenside bzw. amphiphilen Moleküle, genauer eine Kombination aus Blockpolymeren und einer Mischung aus Natrium Acylglutamaten und Alkylpolyglycosiden in einer derartigen Menge eingesetzt, dass sich spontan Mizellen bilden. Diese Mizellen sind Aggregate, die sich aus amphiphilen Molekülen in einem Dispersionsmedium spontan zusammenlagern. Es kann die Vorstellung entwickelt werden, dass der lipophile Teil der Mizelle sich an die unpolaren Stoffe, beispielsweise Make-up-Reste und andere Verunreinigungen, anlagert und diese werden dann mithilfe des hydrophilen Teils der Struktur abgespült bzw. mit dem Reinigungstuch entfernt.

Die Kombination aus wenigstens einem Blockpolymer und einer Mischung aus wenigstens einem Natrium Acylglutamat und wenigstens einem Alkylpolyglycosid, insbesondere die Kombination aus Dinatrium Cocoylglutamat, Decylpolyglucose und Poloxamer 124, liegt mit einem Gehalt von 0,15 bis 10 Gew.-%, bevorzugt 0,36 bis 7 Gew.-%, bezogen auf das Gesamtgewicht des Tränkungsmittels, vor.

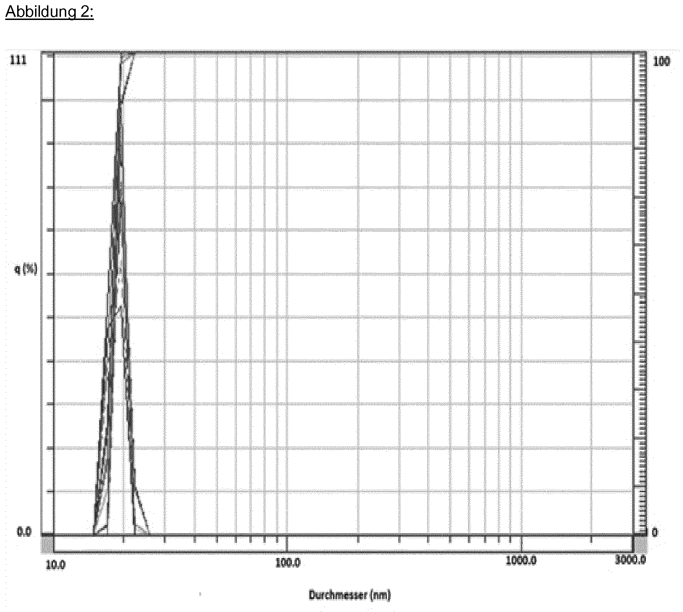

Die Bildung von Mizellen in dem Tränkungsmedium wurde nachgewiesen, siehe dazu Abbildung 2. Beispielhaft wurde eine Kombination aus Dinatrium Cocoylglutamat, Decylpolyglucose und Poloxamer 124, verwirklicht in der ausgewählten Beispielrezeptur 4 mit dem Gerät Horiba LB 550 zur dynamischen Lichtstreuung analysiert. Die Probe wurde als wässrige unverdünnte Probe gemessen. Die Messung erfolgte mit folgenden Parametern:
- Messdauer: 120s
- Anzahl Iterationen: n= 3000
- Art der Verteilung: Anzahl
- Messtemperatur: 23,3°C (Bereich: 23,3-23,4°C),
- Sensor: Flüssigkeitssensor,
- Streulichtintensität (Static): 1,53,
- Streulichtintensität (Dynamic): 3,06.

Auf der x-Achse wurde der Durchmesser der Mizellen angegeben in [nm], auf der y-Achse (links) ist mit q der Anteil einzelner Kurven aufgetragen, auf der rechten Seite die Summe der einzelnen Kurven. Jede einzelne Kurve bedeutet eine Messung, die Vielzahl der Kurven veranschaulicht die Mehrfachmessung. Es konnte die Bildung von Mizellen gezeigt werden. Die gebildeten Mizellen wurden im Bereich von 5 bis 55 nm nachgewiesen.

Das Vorliegen der beschriebenen Mizellen ermöglicht es mithilfe von wässrigen Tränkungsmitteln unpolare Verschmutzungen, insbesondere wasserfeste Make-up-Reste wie wasserfeste Mascara zu entfernen. Das Prinzip liegt in der "Natur der Sache". Mizellen sind so aufgebaut, dass der polare Teil als "Kopfgruppe" nach außen zeigt und sich so als Mizelle im polaren Tränkungsmedium aufhalten kann. Der unpolare Teil der Mizelle liegt innen, sodass der Schmutz direkt in der Mitte eingebunden wird und so mit Hilfe des polaren Teils mit dem Tränkungsmedium abgenommen werden kann.

Dass die Mizellenbildung vorteilhaft für die Produkteigenschaften eines ausgewählten Produkts gemäß der vorliegenden Erfindung ist, zeigte eine Studie mit Probanden. Die Untersuchung wurde an 30 Frauen im Alter von 19 bis 64 Jahren durchgeführt, die nach eigener Einschätzung eine sensible Haut hatten. 7 Tage vor Beginn der Messungen durften die Probanden keine stark Öl-haltigen Hautpflege- und Reinigungsprodukte verwenden, ebenso mussten starke Sonnenexposition und Solarienbesuche vermieden werden. Das Testprodukt wurde in einer üblichen Menge auf die Gesichtshaut aufgetragen, für 7 Tage, zweimal täglich. Das Testprodukt ist in den Beispielrezepturen unter der Nr. 4 angegeben. Die Studienteilnehmer erhielten einen Fragebogen, der am Ende der Studie abgegeben werden musste und ausgewertet wurde. Die Fragen bezogen sich auf verschiedene Produkteigenschaften und Produktleistungen. So wurde u.a. die Reinigungsleistung des Testprodukts abgefragt. 91 % der Probanden gaben an, dass das Testprodukt gründlich reinigt und 82 % urteilten, dass das Testprodukt effektiv Make-up-Reste entfernt. Somit konnte gezeigt werden, dass erfindungsgemäße Tränkungsmittel die Entfernung von Make-up bewirken.

Weiterhin bevorzugt ist der Einsatz von natürlichen, pflanzlichen Ölen in den Tränkungsmitteln. Diese Öle bestehen im Wesentlichen aus gemischten Triacylglycerolen höherer Fettsäuren mit gerader Anzahl von Kohlenstoff-Atomen. Daneben sind geringe Mengen an Acyllipiden (z. B. Sterolester) und ein Anteil an unverseifbaren Bestandteilen enthalten. Pflanzliche Öle weisen praktisch ausschließlich geradzahlige Fettsäuren auf und sind bei 20ºC flüssige Stoffe. Die Fettsäuren können substituiert sein, beispielsweise mit einer oder mehrerer Hydroxylgruppen. Beispiele für erfindungsgemäß einsetzbare pflanzliche Öle sind Mandelöl, Traubenkernöl, Macadamiaöl, Arganöl, Jojobaöl, Avocadoöl, Borrethschöl, Brennnesselsamenöl, Distelöl, Erdnussöl, Hanföl, Kokosöl, Kürbiskernöl, Leinöl, Maiskeimöl, Mohnöl, Nachtkerzenöl, Olivenöl, Sesamöl, Sojaöl, Sonnenblumenkernöl, Walnussöl, Weizenkeimöl, sowie Mischungen hiervon. Besonders bevorzugt sind Mandelöl, Traubenkernöl, Arganöl, Olivenöl, Macadamiaöl sowie Mischungen hiervon.
Ausführungsformen von Tränkungsmitteln, die wenigstens ein natürliches, pflanzliches Öl enthalten, weisen einen Gehalt an natürlichen, pflanzlichen Ölen in den Tränkungsmitteln 0,1 bis 2 Gew.-%, bevorzugt 0,2 bis 1 Gew.-%, bezogen auf das Gesamtgewicht des Tränkungsmittels, auf.
Insgesamt führt die Einarbeitung wenigstens eines natürlichen, pflanzlichen Öls in die erfindungsgemäßen Tränkungsmittel zu einer verbesserten Pflegeleistung und gleichzeitig zu einer Verstärkung der Reinigungsleistung.

Weiterhin vorteilhaft ist die Verwendung von Sorbitol in den Tränkungsmitteln. Sorbitol ist ein Zuckeralkohol und wird in kosmetischen Zubereitungen oft als Feuchthaltemittel eingesetzt. Ursprünglich wurde Sorbitol aus bestimmten Früchten gewonnen, wie beispielsweise aus den Früchten der Eberesche und aus Kernobst. Sorbitol liegt in den Tränkungsmittel mit einem Gehalt von 0,1 bis 5 Gew. %, bevorzugt von 0,25 bis 4 Gew. %, besonders bevorzugt von 0,35 bis 3 Gew.-%, bezogen auf das Gesamtgewicht des Tränkungsmittels, vor. Ausgehend von der Funktion des Sorbitol als Feuchthaltemittel, kann durch die Verwendung von Sorbitol die Befeuchtungsleistung des getränkten Reinigungstuchs insgesamt verbessert werden.

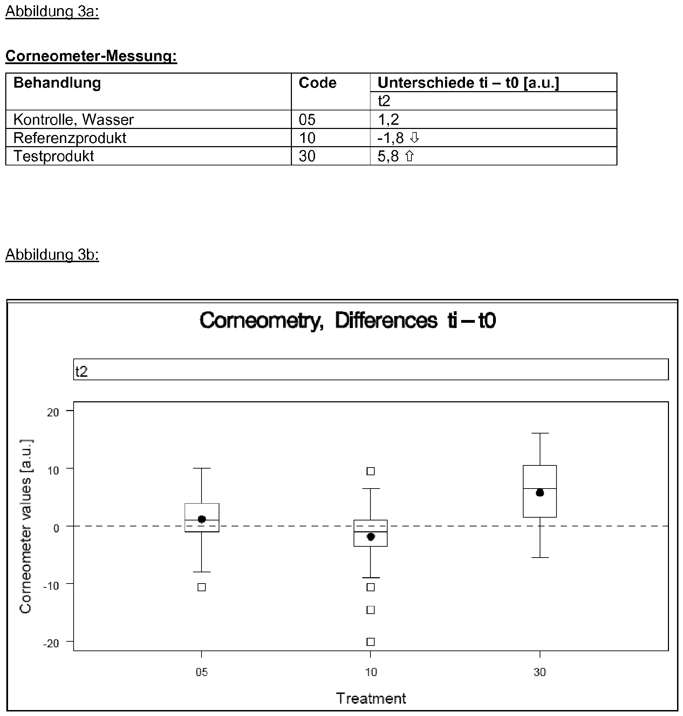

Die Wirkung von Sorbitol wurde untersucht; die Ergebnisse sind in den Abbildungen 3a und 3b dargestellt. Die Untersuchungen wurden an 30 Frauen im Alter von 18 bis 65 Jahren durchgeführt, die nach eigener Einschätzung eine sensible Haut hatten. 7 Tage vor Beginn der Messungen durften die Probanden keine stark Öl-haltigen Hautpflege- und Reinigungsprodukte verwenden, ebenso mussten starke Sonnenexposition und Solarienbesuche vermieden werden. Die erste Messung zur Bestimmung der Hautfeuchte mit dem Corneometer® CM 825 der Firma Courage + Khazaka (Köln, Deutschland) erfolgte nach einer Akklimatisierung von 30 Minuten bei einer Temperatur von 21 ± 1ºC und 50 ± 5 % relativer Luftfeuchtigkeit unter normalen atmosphärischen Bedingungen. Vermessen wurden 3 markierte Areale auf den Unterarmen. Aus diesen Messwerten wurden die Basiswerte der Hautfeuchte bestimmt. Diese Werte entsprechen den Werten zum Zeitpunkt t0. Anschließend wurden auf die 3 Hautareale das Testprodukt, ein Referenzprodukt und als Kontrolle Wasser als Proben aufgetragen. Die Anwendung der jeweiligen Proben erfolgte an 14 Tagen zweimal täglich. Im Anschluss erfolgte eine erneute Corneometermessung der behandelten Hautareale des Unterarms unter identischen Messbedingungen. Die erhaltenen Messwerte entsprechen den Messwerten zum Zeitpunkt t2. Für die Auswertung wurde die Differenz von t2 - t0 gebildet. In der Tabelle und in der Graphik wurden die Mittelwerte der Unterschiede zu den Basiswerten t0 ermittelt und dargestellt. In der Abbildung 3 steht ti für Messzeitpunkte verschieden von t0. Die Einheit a.u. bedeutet arbitrary units. Die Abbildungen zeigen, dass das erfindungsgemäße Produkt eine signifikante Erhöhung der Hautfeuchtigkeit bewirkt (↑). Das Referenzprodukt hingegen führt zu einer signifikanten Verschlechterung der Werte für die Hautfeuchtigkeit (↓).Als Testprodukt diente die Beispielrezeptur Nr. 4. Als Referenzprodukt diente ein interner Standard. Das Testprodukt ist ein ausgewähltes Tränkungsmittel für die Tücher gemäß der vorliegenden Erfindung.

Als Referenzprodukt diente ein internes Standardprodukt, dass neben den Tensiden Disodium Cocoyl Glutamate und Decyl Glucoside, Poloxamer 124, Glycerin, Sorbitol, Propylene Glycol, 1,2-Hexanediol, Trisodium EDTA, Phenoxyethanol und weitere Komponenten in geringeren Mengen enthält.

Weiterhin ist der Einsatz von 1,2-Hexandiol in den erfindungsgemäßen Tränkungsmittel möglich, ohne dass es zu Beeinträchtigungen des Geruchs kommt. Üblicherweise führt der Einsatz von 1,2-Hexandiol in kosmetischen Produkten zu Geruchsbeeinträchtigungen, da 1,2-Hexandiol einen Eigengeruch hat und an sich als geruchlich unangenehm wahrgenommen wird. 1,2-Hexandiol wird eingesetzt um die Befeuchtungsleistung zu unterstützen, das getränkte Tuch feucht zu halten und gleichzeitig die mikrobielle Stabilität der Tücher positiv zu beeinflussen. Der Gehalt von 1,2-Hexandiol in den Tränkungsmitteln beträgt 0,1 bis 1 Gew. %, bevorzugt 0,25 bis 0,5 Gew.-%, bezogen auf das Gesamtgewicht des Tränkungsmittels.

Die Tränkungsmittel können erfindungsgemäß kosmetische Hilfsstoffe enthalten, wie sie üblicherweise in solchen Zubereitungen verwendet werden, z.B. Emulgatoren, Komplexbildner, Parfüme, Farbstoffe. Weitere erfindungsgemäße Hilfsstoffe sind weitere anfeuchtende und/oder feuchthaltende Substanzen, weitere Fette, Öle oder andere übliche Bestandteile einer kosmetischen Formulierung wie Alkohole, Polyole, Polymere, Schaumstabilisatoren, Elektrolyte, organische Lösungsmittel oder Silikonderivate.

Die Probanden, die an der Studie zur Messung der Hautfeuchtigkeit beteiligt waren, haben das Testprodukt auch hinsichtlich der Produkteigenschaften und Produktleistungen beurteilt. Dazu wurde ein Fragebogen verteilt, der am Ende der Studie eingesammelt und ausgewertet wurde. Es zeigte sich, dass das Testprodukt keine Rötungen, keine Spannungsgefühle und keine Trockenheit der Haut verursacht. Zu diesem Urteil kamen 79 % der befragten Probanden. 76 % der befragten Studienteilnehmer urteilten, dass die Anzeichen empfindlicher Haut durch das Testprodukt reduziert werden. Diese Aussagen belegen, dass das Testprodukt, ein ausgewähltes Produkt gemäß der vorliegenden Erfindung, reizarm und verträglich ist.

Kosmetische oder dermatologische Tücher können sowohl aus wasserlöslichen (z. B. wie Toilettenpapier) als auch aus wasserunlöslichen Materialien bestehen. Ferner können die Tücher glatt oder auch oberflächenstrukturiert sein. Erfindungsgemäß bevorzugt sind oberflächenstrukturierte Tücher.

Erfindungsgemäß bevorzugt werden in Kombination mit den erfindungsgemäßen Tränkungsmitteln "trockene" Tücher eingesetzt, welche aus Vlies bestehen, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Werden geprägte Vliese verwendet, so erleichtern große Kavitäten an der Vliesoberfläche und im Vlies die Aufnahme von Schmutz und Verunreinigungen, wenn mit dem getränkten Tuch über die Haut gefahren wird. Die Reinigungswirkung kann gegenüber ungeprägten Tüchern um ein Vielfaches gesteigert werden.

Es hat sich als vorteilhaft herausgestellt für das Tuch, wenn dieses ein Gewicht von 30 bis 120 g/m², vorzugsweise von 35 bis 60 g/m², hat (gemessen bei 20 C ± 2°C und bei einer Feuchtigkeit der Raumluft von 65 % ± 5 % für 24 Stunden).

Die Dicke des Vlieses beträgt vorzugsweise 0,4 mm bis 2 mm, insbesondere 0,45 mm bis 0,7 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % PET und 30 % Tensel/Lyocell. Lyocell ist eine aus Cellulose bestehende, industriell hergestellte Regeneratfaser und weist eine hohe Trocken- und Nassfestigkeit auf. Derartige Tücher sind beispielsweise erhältlich von der Firma Spuntech unter der Bezeichnung 1T3P45P70L30OB.

In einer weiteren besonders vorteilhaften Ausführungsform bestehen die Fasern zu 100% aus Viskose. Derartige Tücher sind beispielsweise erhältlich von der Firma Spuntech unter der Bezeichnung 13P46V100.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilisatoren und/oder Konservierungsmittel enthalten.

Die zur Bildung des Tuches eingesetzten Fasern weisen vorzugsweise eine Wasseraufnahmerate von mehr als 4 mm/[10 sec] (gemessen nach ERT 10.4-02, WSP 10.1), insbesondere mehr als 6 mm/[10 sec] auf.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| im trockenen Zustand | Maschinenrichtung | 15 % bis 100 %, bevorzugt 20 % und 50 % |
| | Querrichtung | 40 % bis 120 %, bevorzugt 50 % und 85 % |
| im getränkten Zustand | Maschinenrichtung | 15 % bis 100 %, bevorzugt 20 % und 40 % |
| | Querrichtung | 40 % bis 120 %, bevorzugt 50 % und 85 % |

Das Gewichtsverhältnis des ungetränkten Tuchs zu dem Tränkungsmittel beträgt erfindungsgemäß vorteilhaft 1:1 bis 1:5.

Gegenstand der vorliegenden Erfindung ist ebenso die Verwendung erfindungsgemäßen Tücher zur Reinigung der Haut, insbesondere der Gesichtshaut.

Auch Gegenstand der vorliegenden Erfindung ist die Verwendung der erfindungsgemäßen Tücher zur Entfernung von dekorativen Kosmetika, insbesondere von wasserfestem Make-up, weiter insbesondere von wasserfester Mascara.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht des Tränkungsmittels bezogen.

**Beispiele:**

| **Inhaltsstoffe** | **1*** | **2*** | **3*** | **4** | **5** | **6*** | **7*** | **8*** |
|---|---|---|---|---|---|---|---|---|
| Aqua | q.s. | q.s. | q.s. | q.s. | q.s. | q.s. | q.s. | q.s. |
| Decyl Glucoside | 0,053 | 0,159 | 0,265 | 0,106 | 0,133 | 0,53 | 0,398 | 0,265 |
| Poloxamer 124 | 0,5 | 0,75 | 2 | 0,25 | 1,5 | 0,5 | 3 | 4 |
| Disodium Cocoyl Glutamate | 0,025 | 0,006 | 0,013 | 0,006 | 0,313 | 0,188 | 0,05 | 0,5 |
| Glyceryl Glucoside | 0,004 | 0,004 | 0,004 | 0,004 | 0,004 | 0,004 | 0,004 | 0,004 |
| Mandelöl | 0 | 0,25 | 0 | 0 | 0 | 0 | 0 | 0 |
| Panthenol | 0,102 | 0,102 | 0,102 | 0,102 | 0,102 | 0,102 | 0,102 | 0,102 |
| Tocopheryl Acetate | 0,058 | 0,058 | 0,058 | 0,058 | 0,058 | 0,058 | 0,058 | 0,058 |
| Traubenkernöl | 0 | 0 | 0 | 0 | 0,1 | 0 | 0 | 0 |
| Polyquaternium-10 | 0,091 | 0,046 | 0,023 | 0,091 | 0,091 | 0,068 | 0,091 | 0,046 |
| PEG-40 Hydrogenated Castor Oil | 0,8 | 1,1 | 1,3 | 1,1 | 1 | 1,5 | 1,5 | 1,6 |
| Arganöl | 0 | 0 | 0 | 0 | 0 | 0 | 0,2 | 0 |
| Parfum | 0,25 | 0,3 | 0,25 | 0,25 | 0,3 | 0,25 | 0,3 | 0,3 |
| Glycerin | 0,748 | 0,995 | 1,49 | 0,5 | 1,985 | 4,955 | 0,995 | 1,49 |
| 1,2-Hexanediol | 0,2 | 0,2 | 0,35 | 0,38 | 0 | 0 | 0,15 | 0 |
| Sorbitol | 0,7 | 1,05 | 0,35 | 0,35 | 1,4 | 2,1 | 0,7 | 0,7 |
| Alcohol Denat. | 0,962 | 3,847 | 1,924 | 3,847 | 1,924 | 0 | 4,809 | 3,847 |
| Citric Acid | 0,06 | 0,06 | 0,06 | 0,06 | 0,06 | 0,06 | 0,06 | 0,06 |
| Propylene Glycol | 0,005 | 0,001 | 0,003 | 0,001 | 0,063 | 0,038 | 0,01 | 0,1 |
| Ethylhexylglycerin | 0 | 0 | 0 | 0,149 | 0,149 | 0,149 | 0 | 0,149 |
| Methylparaben | 0,3 | 0,15 | 0 | 0 | 0,2 | 0,2 | 0 | 0 |
| Phenoxyethanol | 0,5 | 0,4 | 0,6 | 0,6 | 0,6 | 0,6 | 0,6 | 0,4 |
| Benzethonium Chloride | 0 | 0 | 0,04 | 0 | 0 | 0 | 0 | 0,03 |
| Trisodium EDTA | 0,2 | 0,2 | 0,2 | 0,2 | 0,2 | 0,2 | 0,2 | 0,2 |
| Propylparaben | 0 | 0 | 0,1 | 0 | 0 | 0 | 0 | 0 |
| Ethylparaben | 0 | 0,05 | 0 | 0 | 0 | 0 | 0 | 0 |
| Methylpropanediol | 0 | 0 | 0 | 0 | 2 | 2 | 0 | 2 |
| Caprylyl Glycol | 0 | 0 | 0 | 0 | 0 | 0 | 0,2 | 0 |
| Benzyl Alcohol | 0 | 0 | 0 | 0 | 0 | 0 | 0,2 | 0 |
| Piroctone Olamine | 0 | 0 | 0 | 0 | 0,05 | 0 | 0 | 0 |

| | | | | | | | | |
|---|---|---|---|---|---|---|---|---|
| * nicht erfindungsgemäßes Beispiel. | | | | | | | | |

## Patentansprüche

1. Reinigungstücher getränkt mit einem Tränkungsmittel enthaltend wenigstens ein Blockpolymer, eine Mischung aus wenigstens einem Natrium Acylglutamat und wenigstens einem Alkylpolyglycosid und
Alkohol denat.;
wobei das Gewichtsverhältnis von dem wenigstens einen Blockpolymer zu der Mischung aus dem wenigstens einen Natrium Acylglutamat und dem wenigstens einen Alkylpolyglycosid 3,63:1 bis 0,28:1 beträgt.

2. Tücher nach Anspruch 1 **dadurch gekennzeichnet, dass** in dem Tränkungsmittel die Natrium Acylglutamate mit einem Gehalt von 0,001 bis 1 Gew. %, bevorzugt von 0,003 bis 0,75 Gew. %, besonders bevorzugt von 0,005 bis 0,5 Gew. %, bezogen auf das Gesamtgewicht des Tränkungsmittels, vorliegen.

3. Tücher nach wenigstens einem der vorstehenden Ansprüche **dadurch gekennzeichnet, dass** in dem Tränkungsmittel die Alkylpolyglycoside mit einem Gehalt von 0,01 bis 1 Gew. %, bevorzugt von 0,025 bis 0,8 Gew. %, besonders bevorzugt von 0,05 bis 0,6 Gew. %, bezogen auf das Gesamtgewicht des Tränkungsmittels, vorliegen.

4. Tücher nach wenigstens einem der vorstehenden Ansprüche **dadurch gekennzeichnet, dass** in dem Tränkungsmittel das Gewichtsverhältnis von der Natrium Acylglutamate zu den Alkylpolyglycoside 30:1 bis 0,001:1, bevorzugt 20:1 bis 0,05:1 beträgt.

5. Tücher nach wenigstens einem der vorstehenden Ansprüche **dadurch gekennzeichnet, dass** in dem Tränkungsmittel wenigstens ein Blockpolymer, insbesondere Poloxamer 101, Poloxamer 124 und/oder Poloxamer 184, enthalten ist.

6. Tücher nach wenigstens einem der vorstehenden Ansprüche **dadurch gekennzeichnet, dass** in dem Tränkungsmittel das wenigstens eine Blockpolymer mit einem Gehalt von 0,1 bis 8 Gew.-%, bevorzugt von 0,2 bis 5 Gew. %, besonders bevorzugt von 0,25 bis 4 Gew. %, bezogen auf das Gesamtgewicht des Tränkungsmittels, vorliegt.

7. Tücher nach wenigstens einem der vorstehenden Ansprüche **dadurch gekennzeichnet, dass** die Kombination aus wenigstens einem Blockpolymer und einer Mischung aus wenigstens einem Natrium Acylglutamat und wenigstens einem Alkylpolyglycosid mit einem Gehalt von 0,15 bis 10 Gew.-%, bevorzugt 0,36 bis 7 Gew.-%, bezogen auf das Gesamtgewicht des Tränkungsmittels, vorliegt.

8. Tücher nach wenigstens einem der vorstehenden Ansprüche **dadurch gekennzeichnet, dass** in dem Tränkungsmittel wenigstens ein natürliches, pflanzliches Öl enthalten ist.

9. Tücher nach wenigstens einem der vorstehenden Ansprüche **dadurch gekennzeichnet, dass** in dem Tränkungsmittel das wenigstens eine Öl mit einem Gehalt von 0,1 bis 2 Gew. %, bevorzugt von 0,2 bis 1 Gew. %, bezogen auf das Gesamtgewicht des Tränkungsmittels, vorliegt.

10. Tücher nach wenigstens einem der vorstehenden Ansprüche **dadurch gekennzeichnet, dass** in dem Tränkungsmittel Sorbitol enthalten ist.

11. Tücher nach wenigstens einem der vorstehenden Ansprüche **dadurch gekennzeichnet, dass** in dem Tränkungsmittel der Gehalt an Sorbitol von 0,1 bis 5 Gew. %, bevorzugt von 0,25 bis 4 Gew. %, besonders bevorzugt von 0,35 bis 3 Gew. %, bezogen auf das Gesamtgewicht des Tränkungsmittels, beträgt.

12. Tücher nach wenigstens einem der vorstehenden Ansprüche **dadurch gekennzeichnet, dass** die Tücher oberflächenstrukturiert sind.

13. Tücher nach wenigstens einem der vorstehenden Ansprüche, **dadurch gekennzeichnet, dass** das Gewichtsverhältnis von ungetränktem Tuch zum Tränkungsmittel 1:1 bis 1:5 beträgt.

14. Tücher nach wenigstens einem der vorstehenden Ansprüche **dadurch gekennzeichnet, dass** die Tücher aus 100 % Viskose oder einer Mischung aus 70 % PET und 30 % Lyocell bestehen.

15. Verwendung von getränkten Tüchern nach wenigstens einem der vorstehenden Ansprüche zur Reinigung der Haut, insbesondere der Gesichtshaut.

16. Verwendung von getränkten Tüchern nach wenigstens einem der Ansprüche 1 bis 14 zur Entfernung von dekorativen Kosmetika, insbesondere wasserfestem Make-up.

## Claims

1. Cleansing wipes impregnated with an impregnating agent comprising at least one block polymer, a mixture of at least one sodium acyl glutamate and at least one alkyl polyglycoside and
denatured alcohol;
wherein the ratio by weight of the at least one block polymer to the mixture of the at least one sodium acyl glutamate and the at least one alkyl polyglycoside is from 3.63:1 to 0.28:1.

2. Wipes according to Claim 1, **characterized in that** in the impregnating agent, the sodium acyl glutamates are present at a content of 0.001 to 1% by weight, preferably 0.003 to 0.75% by weight, particularly preferably 0.005 to 0.5% by weight, based on the total weight of the impregnating agent.

3. Wipes according to at least one of the preceding claims, **characterized in that** in the impregnating agent, the alkyl polyglycosides are present at a content of 0.01 to 1% by weight, preferably 0.025 to 0.8% by weight, particularly preferably 0.05 to 0.6% by weight, based on the total weight of the impregnating agent.

4. Wipes according to at least one of the preceding claims, **characterized in that** in the impregnating agent, the ratio by weight of the sodium acyl glutamates to the alkyl polyglycosides is from 30:1 to 0.001:1, preferably from 20:1 to 0.05 :1.

5. Wipes according to at least one of the preceding claims, **characterized in that** at least one block polymer is present in the impregnating agent, especially poloxamer 101, poloxamer 124 and/or poloxamer 184.

6. Wipes according to at least one of the preceding claims, **characterized in that** in the impregnating agent, the at least one block polymer is present at a content of 0.1 to 8% by weight, preferably 0.2 to 5% by weight, particularly preferably 0.25 to 4% by weight, based on the total weight of the impregnating agent.

7. Wipes according to at least one of the preceding claims, **characterized in that** the combination of at least one block polymer and a mixture of at least one sodium acyl glutamate and at least one alkyl polyglycoside is present at a content of 0.15 to 10% by weight, preferably 0.36 to 7% by weight, based on the total weight of the impregnating agent.

8. Wipes according to at least one of the preceding claims, **characterized in that** at least one natural vegetable oil is present in the impregnating agent.

9. Wipes according to at least one of the preceding claims, **characterized in that** in the impregnating agent, the at least one oil is present at a content of 0.1 to 2% by weight, preferably 0.2 to 1% by weight, based on the total weight of the impregnating agent.

10. Wipes according to at least one of the preceding claims, **characterized in that** sorbitol is present in the impregnating agent.

11. Wipes according to at least one of the preceding claims, **characterized in that** in the impregnating agent, the sorbitol content is from 0.1 to 5% by weight, preferably from 0.25 to 4% by weight, particularly preferably from 0.35 to 3% by weight, based on the total weight of the impregnating agent.

12. Wipes according to at least one of the preceding claims, **characterized in that** the wipes are surface-structured.

13. Wipes according to at least one of the preceding claims, **characterized in that** the ratio by weight of non-impregnated wipe to the impregnating agent is from 1:1 to 1:5.

14. Wipes according to at least one of the preceding claims, **characterized in that** the wipes consist of 100% viscose or a mixture of 70% PET and 30% lyocell.

15. Use of impregnated wipes according to at least one of the preceding claims for cleansing skin, particularly facial skin.

16. Use of impregnated wipes according to at least one of Claims 1 to 14 for removing decorative cosmetics, especially waterproof make-up.

## Revendications

1. Lingettes de nettoyage imprégnées avec un agent d'imprégnation contenant au moins un polymère séquencé, un mélange d'au moins un glutamate d'acyle sodique et d'au moins un alkylpolyglycoside et de l'alcool dénaturé ;
le rapport en poids entre ledit au moins un polymère séquencé et le mélange dudit au moins un glutamate d'acyle sodique et dudit au moins un alkylpolyglycoside étant de 3,63:1 à 0,28:1.

2. Lingettes selon la revendication 1, **caractérisées en ce que** le glutamate d'acyle sodique est présent dans l'agent d'imprégnation en une teneur de 0,001 à 1 % en poids, de préférence de 0,003 à 0,75 % en poids, de manière particulièrement préférée de 0,005 à 0,5 % en poids, par rapport au poids total de l'agent d'imprégnation.

3. Lingettes selon au moins l'une quelconque des revendications précédentes, **caractérisées en ce que** les alkylpolyglycosides sont présents dans l'agent d'imprégnation en une teneur de 0,01 à 1 % en poids, de préférence de 0,025 à 0,8 % en poids, de manière particulièrement préférée de 0,05 à 0,6 % en poids, par rapport au poids total de l'agent d'imprégnation.

4. Lingettes selon au moins l'une quelconque des revendications précédentes, **caractérisées en ce que** le rapport en poids entre le glutamate d'acyle sodique et les alkylpolyglycosides dans l'agent d'imprégnation est de 30:1 à 0,001:1, de préférence de 20:1 à 0,05:1.

5. Lingettes selon au moins l'une quelconque des revendications précédentes, **caractérisées en ce qu'**au moins un polymère séquencé, notamment le poloxamère 101, le poloxamère 124 et/ou le poloxamère 184, est contenu dans l'agent d'imprégnation.

6. Lingettes selon au moins l'une quelconque des revendications précédentes, **caractérisées en ce que** ledit au moins un polymère séquencé est présent dans l'agent d'imprégnation en une teneur de 0,1 à 8 % en poids, de préférence de 0,2 à 5 % en poids, de manière particulièrement préférée de 0,25 à 4 % en poids, par rapport au poids total de l'agent d'imprégnation.

7. Lingettes selon au moins l'une quelconque des revendications précédentes, **caractérisées en ce que** la combinaison d'au moins un polymère séquencé et d'un mélange d'au moins un glutamate d'acyle sodique et d'au moins un alkylpolyglycoside est présente en une teneur de 0,15 à 10 % en poids, de préférence de 0,36 à 7 % en poids, par rapport au poids total de l'agent d'imprégnation.

8. Lingettes selon au moins l'une quelconque des revendications précédentes, **caractérisées en ce qu'**au moins une huile végétale naturelle est contenue dans l'agent d'imprégnation.

9. Lingettes selon au moins l'une quelconque des revendications précédentes, **caractérisées en ce que** ladite au moins une huile est présente dans l'agent d'imprégnation en une teneur de 0,1 à 2 % en poids, de préférence de 0,2 à 1 % en poids, par rapport au poids total de l'agent d'imprégnation.

10. Lingettes selon au moins l'une quelconque des revendications précédentes, **caractérisées en ce que** du sorbitol est contenu dans l'agent d'imprégnation.

11. Lingettes selon au moins l'une quelconque des revendications précédentes, **caractérisées en ce que** la teneur en sorbitol dans l'agent d'imprégnation est de 0,1 à 5 % en poids, de préférence de 0,25 à 4 % en poids, de manière particulièrement préférée de 0,35 à 3 % en poids, par rapport au poids total de l'agent d'imprégnation.

12. Lingettes selon au moins l'une quelconque des revendications précédentes, **caractérisées en ce que** les lingettes sont structurées en surface.

13. Lingettes selon au moins l'une quelconque des revendications précédentes, **caractérisées en ce que** le rapport en poids entre la lingette non imprégnée et l'agent d'imprégnation est de 1:1 à 1:5.

14. Lingettes selon au moins l'une quelconque des revendications précédentes, **caractérisées en ce que** les lingettes sont constituées par 100 % de viscose ou un mélange de 70 % de PET et 30 % de Lyocell.

15. Utilisation de lingettes imprégnées selon au moins l'une quelconque des revendications précédentes pour le nettoyage de la peau, notamment de la peau du visage.

16. Utilisation de lingettes imprégnées selon au moins l'une quelconque des revendications 1 à 14 pour l'élimination de cosmétiques décoratifs, notamment de maquillage résistant à l'eau.
